# EUROPÄISCHE PATENTSCHRIFT

(11) **EP 2 589 410 B1**
(45) Veröffentlichungstag und Bekanntmachung des Hinweises auf die Patenterteilung: **11.02.2015**
(21) Anmeldenummer: 11008766.5
(22) Anmeldetag: 03.11.2011
(51) Int. Cl.: A61N 1/36, A61B 5/20, A61B 5/0488

(54) **Kontrollanordnung zur intraoperativen Überwachung der Nervenfunktion im Beckenbereich**
Control assembly for intraoperative monitoring of the nerve function in the pelvis
Agencement de contrôle pour la surveillance intraopératoire de la fonction nerveuse dans le bassin

(43) Veröffentlichungstag der Anmeldung: 08.05.2013
(73) Patentinhaber: inomed Medizintechnik GmbH, 79312 Emmendingen (DE)
(72) Erfinder: Krüger, Thilo, 79312 Emmendingen (DE); Somerlik, Karin, 79312 Emmendingen (DE); Mattmüller, Rudi, 79312 Emmendingen (DE); Kneist, Werner, 55116 Mainz (DE); Kauff, Daniel Wilhelm, 55128 Mainz (DE); Lang, Hauke, 55131 Mainz (DE); Koch, Klaus Peter, 66687 Wadern (DE)
(74) Vertreter: Kunst, Manuel Nikolaus Johannes

(56) Entgegenhaltungen:
- EP-A2- 0 366 163
- WO-A1-2011/100162
- US-A- 3 628 538
- US-A1- 2003 100 930

## Beschreibung

Die Erfindung betrifft eine Kontrollanordnung zur im wesentlichen gleichzeitigen Kontrolle der Stimulierbarkeit von durch das vegetative Nervensystem gesteuerten Funktionen im Beckenbereich, welche mindestens jeweils eine Stimulationselektrode und Sensoren zur Messung von Signalen aufweist, und legt deren Verwendung und Methoden, die Kontrollanordnung zu verwenden, dar, wobei auch für derartige Methoden und Verwendungen eingerichtete, insbesondere mit entsprechender Gebrauchsanleitung versehene, Kontrollanordnungen eine Erfindungsverkörperung sind. Die Erfindung wird durch die Ansprüche definiert.

Bei chirurgischen Eingriffen im Bereich des Beckenbodens, beispielsweise bei der Resektion des Enddarmes, besteht ein Risiko, Nervenbahnen zu verletzen, was dazu führen kann, dass die Innervierung der für die Kontrolle des Stuhlgangs und der Blasenentleerung erforderlichen Muskeln unterbrochen wird. Als Folge kann es zu anorektalen, urologischen und sexuellen Dysfunktionen kommen. Damit besteht ein Bedürfnis nach solchen Operationsmethoden, die es erlauben, diese Beeinträchtigungen zu vermeiden oder deren Folgen bereits in der Operationsphase zu vermindern.

Bekannt sind die Beobachtung des intravesikulären Druckes oder die Messung der Schwellung des Penis. Trotz des Vorkommens anorektaler Dysfunktionen infolge von Schädigungen an autonomen Nerven wurde die Erkenntnisgewinnung in Bezug auf den Internen Analen Sphincter (IAS) bisher vernachlässigt. Doch wird der IAS durch die autonomen Nerven (Nerven des autonomen Nervensystems) innerviert.

Beim Neuro-Monitoring (Überwachen der Nervenfunktion) werden gewöhnlich quergestreifte Muskeln (Skelettmuskeln) überprüft, während dies für glatte Muskeln nicht der Fall ist.

Die US 2009/0222058 zeigt die Verwendung einer Vorrichtung, die nicht-invasiv in die Scheide oder den Anus eingeführt werden kann und die das Elektromyogramm (EMG) des Sphincter des Anus oder der Harnblase aufnehmen kann und bei Abfall von dessen Aktivität durch ein automatisch oder durch den Patienten ausgelöstes Signal einen Stimulationsstrom auslöst, der durch die Wand der jeweiligen Körperöffnung wirkt und für die Stimulation des betreffenden Schließmuskels sorgt. Diese Anordnung ist nicht für intraoperative Zwecke geeignet.

Es ist auch bekannt, den Druck der Blase für Überwachungs(= Monitoring)-Zwecke zu messen, beispielsweise mit Drucksensoren wie in US 2003/0100839 A1. Diese Druckschrift sowie auch EP 0 366 163 A2, US 3,628,538 und WO 2011/100162 A1 offenbaren dauerhaft implantierte oder eingeführte therapeutisch wirksame Stimulationseinrichtungen z.B. gegen Inkontinenz. Die Vorrichtungen darin sind jedoch nicht explizit zur Kontrolle der Unversehrtheit von Nerven während einer Operation eingerichtet und es fehlen konkrete Angaben, wie erwähnte EMG-Messignale gemessen werden.

Aufgabe der vorliegenden Erfindung war vor diesem Hintergrund, eine Anordnung zur Verfügung zu stellen, welche einen Anhaltspunkt dafür gibt, ob bei einem chirurgischen Eingriff im Beckenbereich eines Patienten, etwa bei einer Colon- oder Rectum-Resektion, Nerven verletzt werden, durch deren Verletzung die Kontinenz sowohl hinsichtlich der Blase als auch des Anus, und eventuell weiterer Funktionen wie der Sexualfunktionen, in Frage gestellt werden kann. Dies könnte insbesondere auch die Computer-assistierte chirurgische Navigation (Computer assisted surgical navigation) unter Wahrung der Integrität der autonomen Nerven im Becken erleichtern helfen.

Die Aufgabe wird gelöst durch eine eingangs erwähnte Kontrollanordnung, in der die mindestens eine Stimulationselektrode zum intraoperativen Einbringen in den Beckenraum während einer Operation ausgeführt (= eingerichtet oder ausgebildet) ist und die Kontrollanordnung als Sensoren mindestens einen zur Messung der Blasenfunktion (insbesondere Blasendruck, vor allem hydrostatischer Blaseninnendruck) und mindestens einen zur Messung der Aktivität des Sphincter Ani internus aufweist und die Kontrollanordnung zur intraoperativen Kontrolle eingerichtet ist, wobei der mindestens eine Sensor zur Messung der Blasenfunktion jeweils solche zur Messung des Blasendrucks und, als kennzeichnendes Merkmal, der oder die Sensoren zur Messung der Aktivität des Sphincter solche zur EMG-Messung sind, wobei die Aufnahme des EMG-Messsignals durch einen software- und/oder hardware-implementierten Tiefpassfilter auf einen Bereich unter 30 Hz gefiltert wird. Die Einrichtung der Kontrollanordnung zur intraoperativen Kontrolle erfolgt beispielsweise mittels der nachfolgend oder insbesondere in den Beispielen genannten spezifischen Merkmale.

Es wurde überraschend gefunden, dass mittels einer derartigen Vorrichtung die Kontrolle insbesondere der Innervation der Funktion der glatten Muskulatur der Blase und des Sphincter Ani in einfacher Weise ermittelt werden kann, wobei durch die Kombination zusätzlich unterschieden werden kann, welcher Nerv geschädigt wurde, falls dies doch passiert, so dass dann bereits während der Operation geeignete Maßnahmen und Vorkehrungen zu treffen, wie Versuche der Wiederherstellung geschädigter Nerven, Vermeidung weiterer Schädigung oder Anlegung von einem künstlichen Darmausgang, falls die Verbindung zum Spincter ani nicht mehr funktioniert, oder dergleichen.

Das autonome Nervensystem des Beckenbodens beinhaltet den Plexus hypogastricus superior, der Harnblase, Enddarm und Geschlechtsorgane als Bestandteil des Sympathicus versorgt, hypogastrische Nerven (Nn hypogastrici), welche den Beckenbodenbereich von diesem ausgehend versorgen, die Nervi splanchnici lumbales (Pelvic splanchnic nerves, PSN), welche ebenfalls die Blase, Enddarm und Geschlechtsorgane versorgen, und den Plexus hypogastricus Inferior (PHI), der auch Organe im Beckenraum versorgt sowie den Pudendus Nerven.

Im vorliegenden Fall werden durch die erfindungsgemäße Kontrollanordnung die Überwachung des Sphincter anus internus (glatter Teil des Schließmuskels) und des Detrusor Vesicae realisiert.

Der Sphincter anus internus ist ein glatter Muskel, der anders reagiert als die quergestreiften Skelettmuskeln. Daher ist vorzugsweise eine Anpassung der Messvorrichtung dergestalt vorgesehen, dass diese die Ableitung des EMG der glatten Muskeln, insbesondere des Sphincter anus internus, ermöglicht, was am besten bei einer Messung in dem Frequenzbereich von ungefähr 25 oder weniger Hz erfolgt, so dass in einer vorteilhaften Ausgestaltung der erfindungsgemäßen Kontrollanordnung ein entsprechender software- und/oder hardware-implementierter Filter vorgesehen ist.

Was den Detrusor Vesicae angeht, ist die erfindungsgemäße Kontrollanordnung vorzugsweise zur Messung des Druckes vorgesehen, da der Druck innerhalb der Blase ansteigt, wenn der Muskel kontrahiert. Vorteilhaft ist der Druckmesser als Kombination derart ausgeführt, dass der Blasendruck wie ein EMG aufgenommen werden kann, beispielsweise in Form einer Kombination aus einem Drucksensor und vorteilhaft einer dafür eingerichteten separaten Druckmessbox für eine erste Signalnormierung und -weiterleitung.

Die erfindungsgemäße Kontrollanordnung weist vorzugsweise die mindestens eine Stimulationselektrode in Form mindestens einer Sonde für die kontinuierliche (insbesondere elektrische) Stimulation, insbesondere wie im nachfolgenden Beispiel beschrieben und/oder in Form mindestens einer handführbaren Stimulationssonde, insbesondere wie im nachfolgenden Beispiel beschrieben, für eine intermittierende (elektrische) Stimulation, oder diese beiden Sondentypen auf. Denkbar ist auch eine Kombination, welche einen Handgriff zum Platzieren einer (beispielsweise zusammengefalteten) Sonde für kontinuierliche Stimulation beinhaltet, die zum Auffinden geeigneter Orte für die Einbringung der Stimulationsreize, für die intermittierende (während kurzer Abschnitte der Operation) Prüfung während der Operation auf Reizweiterleitung durch die genannten Nerven oder für die kontinuierliche Prüfung geeignet ist, wobei die Sonde für kontinuierliche (während längerer Abschnitte der Operation erfolgende) Stimulation lösbar mit einem Handgriff mit oder ohne Arbeitsteil verbunden ist und dieser Handgriff nach Platzierung der Sonde für die kontinuierliche Stimulation abgenommen werden kann, so dass nur diese Sonde verbleibt.

Außerdem weist sie mindestens einen Sensor zur Messung der Blasenfunktion auf, vorzugsweise in Form eines Drucksensors, der den Druck entweder des Schließmuskels der Blase oder vorzugsweise den hydrostatischen Druck im Inneren der Blase zu messen ausgelegt ist. Hier können sämtliche übliche Drucksensoren, beispielsweise piezoelektrische Sensoren, beinhaltet sein. Vorteilhaft ist eine Kombination aus einem.Drucksensor, gekoppelt an einen Blasenkatheter oder zur direkten Anordnung innerhalb der Blase.

Schließlich weist die Kontrollanordnung mindestens einen Sensor zur Messung der Aktivität des Sphincter, worunter vor- und nachstehend der Sphincter ani internus zu verstehen ist, auf, vorzugsweise in Form einer oder mehrerer, beispielsweise mindestens einer bipolaren, EMG-Ableitelektrode(n), insbesondere wie als EMG-Ableitelektrode im nachfolgenden Beispiel beschrieben. EMG steht für Elektromyogramm.

Vorteilhafterweise sind die genannten Teile der Kontrollanordnung miteinander und mit einem ebenfalls zu dieser Anordnung gehörenden Datenerfassungs- und Stimulierungsmodul verbunden, welches neben einer Anzeigevorrichtung (beispielsweise als Bildschirm, als Overhead-Display (Überkopfanzeige), innerhalb eines optischen Instruments wie einer Brille (oder dergleichen) und optionalen beispielsweise computer- und softwaregestützten Auswertungs- und Dokumentationssausstattungen auch Hard- und/oder Softwarekomponenten zur Aufarbeitung der Messsignale, und außerdem zur Beaufschlagung der Stimulationselektrode(n) mit Stimulationspulsen, aufweist. Die Verbindung kann über geeignete Kabel, aber auch über Funk oder beispielsweise IR erfolgen.

Besonders vorteilhaft werden die Messsignale des jeweils mindestens einen Sensors zur Messung der Blasenfunktion und Sensors zur Messung der Aktivität des Sphincter über eine gemeinsame Box, insbesondere nachfolgend auch als Anschlussbox bezeichnet, dem Datenerfassungs- und Stimulierungsmodul zugeführt. Vorteilhaft ist es dabei, die Messsignale der Sensoren derart auszugestalten, dass sie beide wie ein EMG-Messsignal angezeigt werden können, was durch die entsprechende Ausstattung der Anschlussbox ermöglicht werden kann. So kann besonders vorteilhaft eine einzige - gegebenenfalls auch in das Datenerfassungs- und Stimulierungsmodul integrierte - Anschlussbox verwendet werden, die - insbesondere wie in dem nachfolgenden Beispiel beschrieben - derart ausgestattet ist, dass sie ein Drucksignal genauso weiterleiten kann wie ein EMG-Messsignal. Somit ist es möglich auf die Verwendung von zwei separaten Messboxen zu verzichten. Dies kann beispielsweise mittels der in den Beispielen genannten Merkmale erfolgen, insbesondere mittels eines modulierten Messsignals für den Drucksensor, beispielsweise moduliert als Wechselspannung, beispielsweise mit einer Frequenz von 100 bis 10000 Hz, z.B. von ungefähr 500 bis 2000 Hz.

Alternativ kann die Anschlussbox für die EMG-Messsignale und für den Blasendruck auch durch eine separate Druckmessbox zur Überwachung und Weiterleitung der blasenspezifischen Messdaten und durch eine separate Elektroden-Minibox zur Überwachung und Weiterleitung der EMG-Messsignale ersetzt sein.

Vorteilhaft ist die erfindungsgemäße Kontrollanordnung derart ausgestattet, dass die Stimulationssignale, welche beispielsweise über das Kabel für die Stimulation zugeleitet werden können, und die EMG-Signale aufeinander abgestimmt sind, um eine gegenseitige Störung zu verhindern. Gleichzeitig liegt (im Gegensatz zu quergestreifter Muskulatur, wo bereits mit beispielsweise 3 Hz eine Stimulierung möglich ist) die Frequenz für die Stimulierung des glatten Muskels (wie des Sphincter ani internus oder des Detrusor vesicae) bei ungefähr 20 Hz oder höher und liegt idealerweise über 30 Hz. Das (beispielsweise mit der bipolaren EMG-Ableitelekrode ableitbare) EMG-Signal des glatten Muskels bei Stimulation wird daher die Aufnahme des Messsignals auf einen Bereich unter 30 Hz, vorzugsweise von 25 Hz oder niedriger, durch entsprechende Filterung mittels eines Tiefpassfilters, beispielsweise in der Anschlussbox oder dem Datenerfassungs- und Stimulierungsmodul, beispielsweise zwischen ungefähr 0,5 (vorzugsweise ungefähr 5) bis ungefähr 25 Hz, gefiltert, um so eine Störung und Hervorrufung von Messartefakten durch das Stimulierungssignal zu vermindern oder zu vermeiden, denn die Hauptfrequenzkomponenten des glatten Muskels liegen im Bereich unter ungefähr 25 Hz.

Umgekehrt kann, sofern auch das EMG des quergestreiften Muskels aufgenommen werden soll, eine andere geeignete Filterung und Stimulierungsfrequenz vorgesehen sein. Die entsprechenden Messzyklen für glatten und quergestreiften Muskel können auch in geeigneter Reihenfolge abwechselnd vorgesehen sein.

Beispielsweise kann die Verstärkung für das EMG-Signal des Sphincter internus ani 100 bis 5000-fach sein, beispielsweise ungefähr 2500-fach, und die Grenzfrequenz bei 0,01 Hz oder 5 Hz für den Hochpassfilter eingestellt werden, während die Filtereinstellungen für den Sphincter externus ani auch eine Verstärkung im angegebenen Bereich und eine Grenzfrequenz von 30 Hz für den Hochpassfilter aufweisen können.

So ist eine genaue zeitliche Korrelation zwischen Stimulation, EMG-Antwortsignalen und Blasendruck durch gleichzeitige Signalweiterleitung möglich.

Für die Stimulierung werden beispielsweise Ströme zwischen 1 mA und 15 mA, z.B. zwischen 8 oder 12 mA, gewählt. Die Stimulierung kann beispielsweise bei ungefähr 30 Hz mit beispielsweise rechteckigen einphasigen Pulsen mit einer Pulsdauer von ungefähr 200 µs erfolgen. Bei der intermittierenden Stimulation sind beispielsweise Pulszüge von 5 bis 30 Sekunden möglich, mit geeigneten Intervallen von beispielsweise 10 bis 600 Sekunden, z.B. ungefähr 60 bis 120 Sekunden.

"Intraoperativ" bedeutet, dass die erfindungsgemäße Kontrollanordnung während einer Operation, insbesondere zur Kontrolle der Integrität von Nerven (vor allem des autonomen Nervensystems im Beckenbereich) verwendet werden kann, also entsprechend ausgestattet ist, das heißt, die Komponenten sind insbesondere nicht zum postoperativen Verbleib im Patienten ausgelegt, somit handelt es sich also nicht um (für längere Zeit postoperativ im Patienten verbleibende) Implantate, und die Stimulationselektroden sind insbesondere auch nicht zur Einleitung (bei fehlender Kontrolle über die Entleerung) oder Unterbindung (im Falle von Inkontinenz) von Darm- und Blasenentleerung eingerichtet und/oder einzubringen. Die Einführung der Kontrolleinrichtungen ist insbesondere selbst nicht Ziel der Operation, sondern sie dienen zur Kontrolle der Nervenintegrität bei Operationen mit anderen Zielen, wie beispielsweise bei vor- und nachstehend erwähnten Operationen. So kann die Vorrichtung insbesondere verwendet werden, um zu vermeiden, Nervenbahnen zu verletzen, was dazu führen kann, dass die Innervierung der für die Kontrolle des Stuhlgangs und der Blasenentleerung erforderlichen Muskeln unterbrochen wird, so dass es nicht zu anorektalen, urologischen und sexuellen Dysfunktionen kommt, oder, falls doch Nervenbahnen verletzt werden, zum intraoperativen Vornehmen von Maßnahmen zur Verminderung der negativen Folgen oder zur Vorbereitung geeigneter weiterer derartiger Maßnahmen.

Zu den mit der erfindungsgemäßen Kontrollanordnung durchführbaren Methoden und Verwendungen ist anzumerken, dass dabei die erfindungsgemäße Kontrollanordnung intraoperativ zur Kontrolle der Funktionsfähigkeit von Nerven im Beckenbereich eines Patienten insbesondere des autonomen Nervensystems verwendet wird, wobei eine - z.B. gleichzeitige oder parallele oder abwechselnde - Messung der Blasenfunktion und der Funktion des Sphincter ani internus eine Kontrolle der Unversehrtheit von Nerven des autonomen Nervensystems im Beckenraum nach deren Reizung mittels der einen oder mehreren Stimulationselektroden ermöglicht.

Bei einer Operation kann mindestens eine Stimulationselektrode, beispielsweise eine handführbare Stimulationssonde, im Operationsbereich vom Chirurgen so platziert werden, bis ein geeignetes Messsignal für Blasen- und/oder Sphincterfunktion im Datenerfassungs- und Stimulierungsmodul erkennbar wird. Dann oder alternativ direkt kann eine Sonde für kontinuierliche Stimulation an der so gefundenen Stelle platziert werden, was eine dauerhafte (kontinuierliche) Messung ermöglicht. Dies zeigt an, dass ein geeigneter Nervenbereich gefunden wurde. An dieser Stelle kann dann für die kontinuierliche Messung eine Sonde für kontinuierliche Stimulation platziert werden. Gleichzeitig oder alternierend kann mittels des jeweils mindestens einen oder mehreren Sensoren für die Messung der Blasenfunktion (Blasendruck, vor allem hydrostatischer Blaseninnendruck) und zur Messung der Aktivität des Sphincter (EMG) die Überprüfung der Integrität der vor allem autonomen Nerven im Beckenbodenraum, insbesondere wie sie eingangs oder insbesondere im nachfolgenden Beispiel genannt sind, erfolgen.

Werden Nerven des autonomen Nervensystems geschädigt, so wird dies für den Chirurgen erkennbar an der Abschwächung oder dem Fehlen von Messsignalen der - jeweils mindestens einen - Sensoren für die Messung der Blasenfunktion, die Messung der Aktivität des Sphincter oder beide nach (elektrischer) Stimulation. Je nachdem, welches Signal abgeschwächt wird und/oder ab welchem Stimulationspunkt eine Stimulation nicht weitergeleitet wird, kann so auch auf die Art der Schädigung und/oder die Art des geschädigten Nervs rückgeschlossen werden. So ergeben sich durch die Kombination der Sensoren Informationen, die über die Verwendung nur jeweils eines Typs davon hinausgehen, weil aus der Art der Ausfälle genauer auf die Art der Schädigung und den oder die geschädigten Nerven zurückgeschlossen werden kann.

Mittels der erfindungsgemäßen Kontrollanordnung zugängliche Methoden und Verwendungen finden sich in den Beispielen.

"Ungefähr" vor Zahlenwerten bedeutet stets, dass die entsprechende genannte Zahl um einen Streubereich abweichen kann, beispielsweise um ± 20 %, ± 10 % oder insbesondere ± 5 %. "Ungefähr" vor Zahlenwerten kann auch weggelassen werden.

"Im wesentlichen gleichzeitig" bedeutet, dass die verschiedenen Messsignale mindestens alle während einer Operation aufgenommen werden können, beispielsweise simultan, alternierend oder mit Pausen dazwischen.

Die nachfolgenden Figurbeschreibungen stellen mögliche Beispiele für erfindungsgemäße Ausführungsformen dar.

Es zeigt:
- Fig.1:: Erfindungsgemäße Kontrollanordnung in grobschematischer Übersicht - Gesamtaufbau eines Neuromonitoring (= Neuroüberwachungs)-Systems für die Überwachung der Integrität von Nervenstrukturen im kleinen Becken. Das System bietet insbesondere die Möglichkeit der Messaufnahme und Stimulation sowie der Darstellung der erhaltenen Signale. Angeschlossen sind hier eine EMG-Messmodalität und eine Druckmessung. Für die Stimulierung kann eine handgeführte und/oder eine platzierte kontinuierliche Sonde verwendet werden.
- Fig.2:: Schematische Darstellung einer bipolaren Ableitelektrode für Elektromyogramme (EMG) insbesondere des Signals des inneren Schließmuskels des Anus, wie sie für eine erfindungsgemäße Kontrollanordnung Einsatz finden kann.
- Fig.3:: Schematische Darstellung einer handführbaren Stimulations-Elektrode für intermittierende Stimulation als Teil einer Kontrollanordnung gemäß Fig. 1 - diese ermöglicht die einfache Identifizierung von Nerven im Situsbereich.
- Fig.4:: Schematische Darstellung einer möglichen Ausführungsform einer kontinuierlich einsetzbaren Stimulationselektrode als Teil einer Kontrollanordnung gemäß Fig. 1. Diese kann im Situsbereich platziert sein und dort während der Operation verbleiben, was eine kontinuierliche Stimulation ermöglicht.
- Fig.5:: Schematische Darstellung des druckmessenden Anteils einer Kontrollanordnung gemäß Fig. 1.

Mögliche bevorzugte Ausführungsformen der Erfindung finden sich in den nachfolgenden Beispielen, wie auch in den Ansprüchen, die hier durch Bezugnahme aufgenommen werden.

### Beispiele

Die nachfolgenden Beispiele für erfindungsgemäße Kontrollanordnungen dienen der Illustration der Erfindung, ohne ihren Umfang einzuschränken, wobei einzelne oder mehrere bis alle im Beispiel präzisierten Merkmale unabhängig voneinander anstelle von vor- und nachstehenden breiter definierten entsprechenden Merkmalen in den entsprechenden Ausführungsformen eingesetzt werden können und so besondere Ausführungsformen der Erfindung definieren. Die Kontrollanordnungen ermöglichen die Durchführung der unten (und allgemein oben) dargelegten Methoden und Verwendungen.

### Beispiel 1: Kontrollanordung

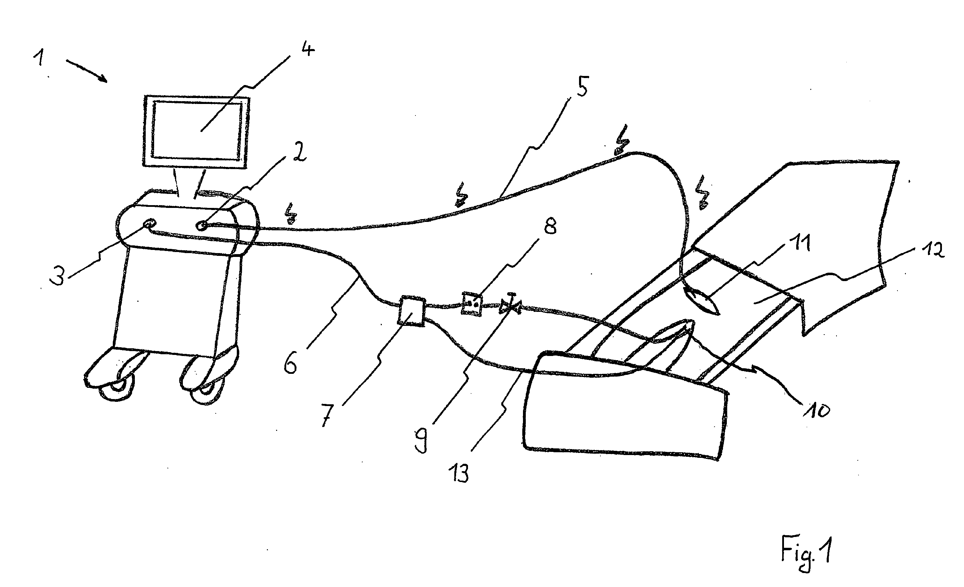

In Figur 1 ist exemplarisch eine erfindungsgemäße Kontrollanordnung **1** dargestellt, welche, neben möglichen weiteren nicht gezeigten Anschlüssen einen Anschluss für eine Stimulationsableitung **2** zu einem (schematisch im Beckenbereich gezeigten) Patienten **12**, welche Stimulationssignale abgeben kann, und ein Anschluss für eine Messsignaleinleitung **3** in ein schematisch dargestelltes Datenerfassungs- und Stimulierungsmodul **4**, welches eine (hier als Bildschirm dargestellte) Anzeigevorrichtung aufweist.

Ein Kabel für Stimulationssignale **5** (also deren Leitung, z.B. als elektrische Signale) ist zur Übermittlung von derartigen Signalen von der Stimulationsleitung **2** in den geöffneten Abdomen des Patienten **12** vorgesehen.

Die Messsignale aus dem Patienten können über ein zusammenfassendes Kabel für eine gebündelte Zuleitung der (beispielsweise normierten) Messsignale dem Datenerfassungs- und Stimulierungsmodul **4** zugeführt werden.

Eine Anschlussbox **7** als Messumformer, die vorteilhaft derart ausgestaltet ist, dass sie ein Drucksignal genauso aufnehmen und weiterleiten kann wie ein Elektromyogramm-Messsignal (EMG-Signal) aus einem Messaufnehmer, ist mit dem Kabel für gebündelte Zuleitung der Messsignale **6** verbunden. In die Anschlussbox **7** führen einerseits Messsignale aus einem Drucksensor **8,** der direkt im Blasenbereich eingebracht oder, wie in Fig. 1 gezeigt, über einen Katheter, der optional ein Ventil **9** aufweist, mit dem Blaseninneren verbunden sein kann und andererseits Messsignale aus einer Ableitung für EMG-Signale **13,** bevor die entsprechenden (gegebenenfalls normierten) Signale an das Datenerfassungs- und Stimulierungsmodul **4** weitergeleitet werden. Die entsprechenden Messaufnehmer sind im Platzierungsbereich der Messaufnehmer **10** vorgesehen, beispielsweise ein Katheter in die Blase oder eine EMG-Ableitungsvorrichtung am Sphincter des Anus des Patienten **12,** während die Stimulationselektrode(n) innerhalb einer Operationsöffnung **11** angeordnet werden.

Anstelle der gezeigten Kabel **5** und **6** sowie **13** und anderer Verbindungen kann auch eine über elektromagnetische Wellen, wie Funksignale oder Licht, vermittelte Übertragung vorgesehen sein - dann kann beispielsweise der Stimulator näher beim Patienten zu liegen kommen und das Datenerfassungs- und Stimulierungsmodul **4** drahtlos mit den übrigen Komponenten verbunden sein, wie auch einzelne oder mehrere dieser Komponenten untereinander.

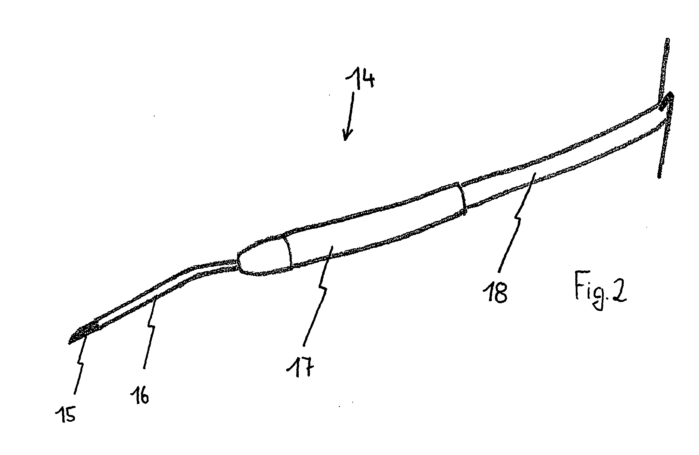

Als Messaufnehmer für das EMG-Signal eignet sich beispielsweise eine bipolare EMG-Ableitelektrode **14**, wie in Fig. 2 dargestellt. Diese weist einen ersten Pol **15** und konzentrisch darum und von dem ersten Pol **15** elektrisch isoliert einen zweiten Pol **16** auf. Sie kann auch einen Handgriff **17** aufweisen und ist hier mit einem Leitungskabel **18** dargestellt, welches eine Verbindung zur Anschlussbox **7** erlaubt. Die bipolare Ableitelektrode **14** eignet sich zur Platzierung in glatter Muskulatur wie dem Sphincter Ani internus, etwa durch Eintreiben. Alternativ können auch zwei einpolige Elektroden verwendet werden, oder Elektroden mit mehr als zwei Polen. Die bipolare Ableitelektrode **14** kann beispielsweise einen Durchmesser von ungefähr 0,1 bis ungefähr 1 mm haben, z.B. ungefähr 0,5 mm, und eine Länge von ungefähr 5 bis ungefähr 30 mm, z.B. ungefähr 15 bis ungefähr 25 mm.

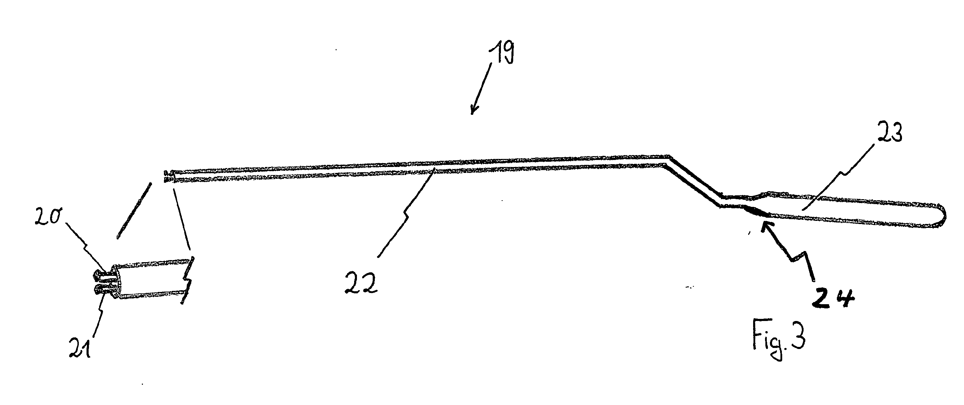

Für die Stimulation kann eine handführbare Stimulationssonde **19**, wie in Fig. 3 dargestellt, die beispielsweise einen ersten (wo von Polen die Rede ist, elektrischen) Pol **20** und einen zweiten Pol **21** aufweisen kann (deren Abstand von den einander am nächsten kommenden Seiten aus gesehen beispielsweise 1 bis 10 mm betragen kann), wobei diese Pole vorzugsweise eine sehr glatte Oberfläche ohne scharfe Kanten aufweisen, um Verletzungen beim Einführen zu vermeiden, vorgesehen sein. Diese kann derart ausgestaltet sein, dass sie einen Arbeitsteil **22** aufweist, der in die Operationswunde eingeführt werden kann, und einen Handgriff **23**, wobei Arbeitsteil und Handgriff auch ohne klare Grenze ineinander übergehen können. Diese handführbare Stimulationselektrode **19** ermöglicht einerseits, geeignete Stellen für die Stimulation im Operationsbereich zu finden, über welche Stimulationen der Beckennerven an den Sphincter des Anus und die Blase, dort insbesondere den Detrusor vesicae, weitergeleitet werden, um so geeignete Stimulationsstellen zu finden. Andererseits ermöglicht sie die intermittierende Prüfung während der Operation, ob Stimulationsreize noch über die Beckennerven an den Sphincter des Anus und die Blase, insbesondere den Detrusor vesicae, weitergeleitet werden. Die Weiterleitung der Reize resultiert jeweils in entsprechenden EMG- Signalen und Druckmesssignalen, die eine Reaktion der glatten Blasenmuskulatur und/oder der glatten (ggf. auch der quergestreiften) Muskulatur des Anus sphincter internus (glatt) bzw. externus (quergestreift) anzeigen. Reagiert eines oder beide der angesprochenen Zielorgane nicht mehr, ist dies beim Suchen nach geeigneten Stimulationsstellen ein Hinweis darauf, dass die geeignete Stimulationsstelle noch nicht gefunden oder durch unbeabsichtigtes Verschieben verloren gegangen ist, oder hier oder im Falle der intermittierenden Prüfung ein Hinweis auf eine mögliche unbeabsichtigte Nervenläsion.

Die handführbare Stimulationselektrode **19** kann einen Anschlussbereich **24** aufweisen (beispielsweise in Form geeigneter Steckkontakte), der beispielsweise die lösbare Verbindung mit einem Kabel für Stimulation **5** ermöglicht - der Vorteil ist hier, dass die Elektrode leicht ausgetauscht werden kann. Alternativ kann das Kabel direkt angeschlossen sein.

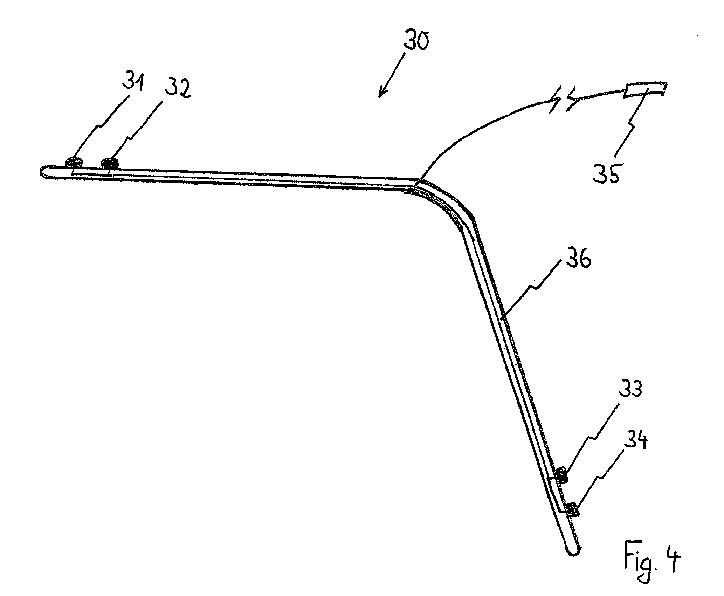

Für eine kontinuierliche Prüfung während der Operation, ob Stimulationsreize noch über die Beckennerven an den Sphincter des Anus und die Blase, insbesondere den Detrusor vesicae, weitergeleitet werden, kann eine Sonde für kontinuierliche Stimulation **30,** für die eine beispielhafte Ausführungsform in Fig. 4 gezeigt ist, eingesetzt werden. Die Ausführungsform in Fig. 4 weist beispielsweise zwei Elektrodenpaare, die jeweils einen Pol 1 **31**, **33** und einen Pol 2 **32**, **34** tragen, auf. Alternativ könnten auch nur ein Elektrodenpaar oder mehr als zwei Elektrodenpaare vorliegen. Die Elektrode kann, wie in Fig. 4 gezeigt, die in einer möglichen vorteilhaften Ausführungsform die zuführenden Leitungen **36** in einem federnd elastischen Material, beispielsweise aus einem Kunststoff mit oder ohne Verstärkungselemente, wie elastischen Draht oder dergleichen, beinhalten, wie in Fig. 4 in Form eines elastischen flexiblen Trägermaterials mit darin enthaltenen Leitungen zu den Elektrodenpaaren **37** schematisch angedeutet. So kann sie durch Zusammenfaltung in Richtung des gezeigten kleineren Winkels (mit den Elektroden nach außen) in den Operationsbereich eingeführt werden und spreizt sich nach dem Loslassen auf, so dass die Elektroden an die Wände des Operationsbereichs (Platzierungsbereich der Messaufnehmer **10**) gepresst und die Sonde für kontinuierliche Stimulation **30** auf diese Weise hinreichend fest verankert wird. Ein Verbindungsstück **35** kann vorgesehen sein (beispielsweise in Form geeigneter Steckkontakte), das die lösbare Verbindung mit einem Kabel für Stimulation **5** ermöglicht - der Vorteil ist hier, dass die Elektrode leicht ausgetauscht werden kann. Alternativ kann das Kabel direkt angeschlossen sein.

Denkbar ist auch eine Kombination, welche einen Handgriff zum Platzieren einer (beispielsweise zusammengefalteten) Sonde für kontinuierliche Stimulation **30** beinhaltet, die zum Auffinden geeigneter Orte für die Einbringung der Stimulationsreize, für die intermittierende Prüfung während der Operation auf Reizweiterleitung durch die genannten Nerven oder für die kontinuierliche Prüfung darauf geeignet ist, wobei die Sonde für kontinuierliche Stimulation **30** lösbar mit einem Handgriff mit oder ohne Arbeitsteil verbunden ist und dieser Handgriff nach Platzierung der Sonde für die kontinuierliche Stimulation 30 abgenommen werden kann, so dass nur diese Sonde verbleibt.

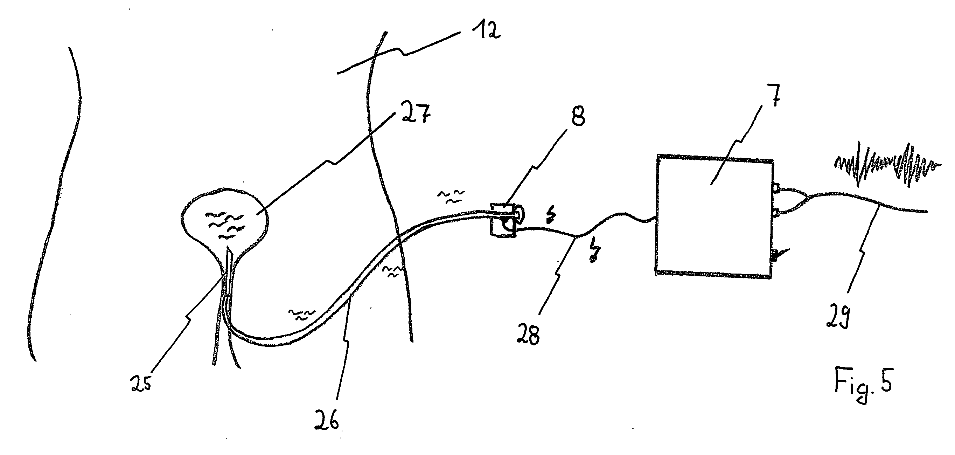

Ein Beispiel für den Teil einer erfindungsgemäßen Kontrollanordnung **1**, der für eine Messung des Drucks in der Blase geeignet ist, wobei ein Druckanstieg nach Nervenstimulation anzeigt, dass die Nervenbahnen zum Detrusor vesicae noch funktionieren, zeigt Fig. 5. So ist dort beispielsweise ein Blasenkatheter **25** vorgesehen, der in der hier gezeigten Ausführungsform (hier hydraulisch) über einen Katheterschlauch **26** mit einem (beispielsweise handelsüblichen, wie Piezo-) Drucksensor **8** als Aufnehmer verbunden ist und in diesen den Blasendruck einleitet. Über eine (gegebenenfalls an einem oder beiden Enden lösbar verbundene) elektrische Verbindung **28** gelangt das elektrische Messsignal in die Anschlussbox **7** und das - gegebenenfalls normierte - elektrische Messsignal wird über das oder die - gegebenenfalls lösbar verbundene (n) - Leitungen des (gegebenenfalls normierten) Messsignals **29** an das in Fig. 1 gezeigte Datenerfassungs- und Stimulierungsmodul **4** weitergeleitet.

Alternative Varianten sind denkbar - beispielsweise kann die Anschlussbox **7** auch in das Datenerfassungs- und Stimulierungsmodul **4** integriert vorgesehen sein.

Vorteilhaft sind die Stimulationssignale, welche beispielsweise über das Kabel für die Stimulation zugeleitet werden können, und die EMG-Signale aufeinander abgestimmt, um eine gegenseitige Störung zu verhindern. Gleichzeitig liegt (im Gegensatz zu quergestreifter Muskulatur, wo bereits mit beispielsweise 3 Hz eine Stimulierung möglich ist) die Frequenz für die Stimulierung des glatten Muskels (wie des Sphincter ani internus oder des Detrusor vesicae) bei ungefähr 30 Hz oder höher. Das (beispielsweise mit der bipolaren EMG-Ableitelektrode **14)** ableitbare EMG-Signal bei Stimulation glatten Muskels wird daher vorzugsweise auf einen Bereich unter 30 Hz, vorzugsweise von 25 Hz oder niedriger, durch entsprechende Filterung mittels eines Tiefpassfilters, beispielsweise in der Anschlussbox **7** oder dem Datenerfassungs- und Simulierungsmodul **4**, beispielsweise zwischen ungefähr 0,5 (vorzugsweise ungefähr 5) bis ungefähr 25 Hz, gefiltert, um so eine Störung und Hervorrufung von Messartefakten durch das Stimulierungssignal zu vermindern oder zu vermeiden, denn die Hauptfrequenzkomponenten des glatten Muskels liegen im Bereich unter ungefähr 25 Hz.

Umgekehrt kann, sofern auch das EMG des quergestreiften Muskels aufgenommen werden soll, eine andere geeignete Filterung und Stimulierungsfrequenz vorgesehen sein. Die entsprechenden Messzyklen für glatten und quergestreiften Muskel können auch in geeigneter Reihenfolge abwechselnd vorgesehen sein.

Beispielsweise kann die Verstärkung für das EMG-Signal des Sphincter internus ani 100 bis 5000-fach sein, beispielsweise ungefähr 2500-fach, und die Grenzfrequenz bei 0,01 Hz oder 5 Hz für den Hochpassfilter eingestellt werden, während die Filtereinstellungen für den Sphincter externus ani auch eine Verstärkung im angegebenen Bereich und eine Grenzfrequenz von 30 Hz für den Hochpassfilter aufweisen können.

Für die Stimulierung werden beispielsweise Ströme zwischen 1 mA und 15 mA, z.B. zwischen 8 oder 12 mA, gewählt. Die Stimulierung kann beispielsweise bei ungefähr 30 Hz mit beispielsweise rechteckigen einphasigen rechtwinkligen Pulsen mit einer Pulsdauer von ungefähr 200 µs erfolgen. Bei der intermittierenden Stimulation sind beispielsweise Pulszüge von 5 bis 30 Sekunden möglich, mit geeigneten Intervallen von beispielsweise 10 bis 600 Sekunden, z.B. ungefähr 60 bis 120 Sekunden.

Es kann auch vorgesehen sein, dass mittels entsprechender Hard- und/oder Softwareausstattung die mittlere Amplitude des aufgenommenen EMG-Signals des glatten Muskels errechnet und angezeigt wird, was dem Chirurgen eine Ja/Nein-Information bietet, so dass er einfach entscheiden kann, ob die Stimulation erfolgreich ist oder nicht.

Die Anschlussbox **7** erlaubt zugleich die Aufnahme des Messsignals der bipolaren EMG-Ableitelektrode(n) **14** und des Drucksensors **8**. Vorteilhaft ist die Anzeigeeinheit des Darstellungs- und Stimulierungsmoduls **4** derart ausgestaltet, dass das Drucksignal des Drucksensors **8** in cm Wassersäule angezeigt wird, doch kann es auch einfach als Spannungssignal angezeigt werden.

Die Druckmessung über den Drucksensor **8** erfolgt vorzugsweise über ein hochfrequentes Wechselstromsignal (beispielsweise von ungefähr 200 bis ungefähr 5000 Hz, z.B. ungefähr 1 kHz), was besonders gut ermöglicht, dass das Signal des EMG-Verstärkers und das des Drucksensors gleichzeitig verarbeitet werden können, beispielsweise über die Anschlussbox **7**. Dank dieser Anpassung kann diese beide Signale an das Darstellungs- und Stimulierungsmodul **4** weiterleiten, wo das Signal genau wie ein freilaufendes EMG-Signal aufgezeichnet werden kann. So ist eine genaue zeitliche Korrelation zwischen Stimulation, EMG-Antwortsignalen und Blasendruck gegeben. Unterschiede des Drucks im Inneren der Blase bewirken eine Amplitudenänderung des Messsignals und können so leicht erkannt werden.

Geeignete Gerätekomponenten für die Kontrollanordnung können beispielsweise folgende sein, wobei andere äquivalente Komponenten nicht ausgeschlossen sein sollen:
Handsonde 522 610 oder vergleichbare bipolare Sonden wie z.B. 522027 der Firma inomed Medizintechnik GmbH, Teningen, Deutschland (nachfolgend "inomed") als handführbare Stimulationssonde **19**.
Konstantstromstimulator (Constant Current Stimulator) der Firma inomed, z,B. im Bereich von 0,05 mA bis 15 mA in 0,1 mA Schritten, max. +/- 30 V Stimulationsspannung Bipolare Nadelelektrode 530 666 (inomed) als Bipolare EMG-Ableitelektrode **14.**
NeMo mit Software "Neuro Explorer" jeweils für das Datenerfassungs- und Stimulierungsmodul **4** (inomed) mit entsprechendem Verstärkerteil.
Blasendruckbox EW0190 der Firma inomed als Anschlussbox **7.** Druckumwandler, inomed, als Drucksensor **8**.

Für die Druckmessung kann beispielsweise folgende Anordnung und Methoden/Verwendungen gewählt werden:
Durch ein Verbindungsstück von Stufenkegel auf Luer-Lock wird ein Katheter über einen Drei-Wege-Hahn mit dem Drucksensor **8** verbunden. An das noch freie Ende des Drei-Wege-Hahns wird ein Schlauch angeschlossen, der lang genug ist, um aus dem sterilen OP-Bereich geführt zu werden. Der Drucksensor **8** selbst sollte nach Möglichkeit auf der gleichen Höhe liegen wie die Blase. An das distale Ende des Schlauches wird ein zweiter Drei-Wege-Hahn angeschlossen, an den während einer Operation ein Urinbeutel angeschlossen werden kann (beispielsweise über ein Luer-Lock). Über den dritten Zugang des Drei-Wege-Hahns kann eine Spritze angeschlossen werden.

Vor der Operation sollte das ganze Schlauchsystem mit einer geeigneten Lösung (z.B. Ringer-Lactat oder physiologische Kochsalzlösung) - unbedingt blasenfrei - gefüllt werden. Dazu können der Drucksensor **8** und der Urinbeutel vom restlichen System mittels des Drei-Wege-Hahns abgekoppelt werden und der Katheter gefüllt werden. Dann werden der Katheter und der Urinbeutel abgeklemmt und der Drucksensor **8** (beispielsweise ein handelsüblicher piezoelektrischer Drucksensor) mit der Lösung ebenfalls blasenfrei gefüllt und dessen Ausgang dann mit einem Stopfen verschlossen.

Während der Operation wird dann der Wegehahn zum Blasenkatheter so gestellt, dass alle Ausgänge offen sind. während der Hahn zum Urinbeutel zunächst so eingestellt wird, dass der Urinbeutel angeschlossen und die Spritze abgeklemmt ist. Während der kritischen Phase, in der die Stimulation erfolgen soll, wird dann zunächst der Urinbeutel abgeklemmt und mit Hilfe der Spritze die Blase mit z.B. 40 bis 100 ml Lösung gefüllt (ggf. Blase erst entleeren und dann befüllen, um definiertes Volumen zu ermöglichen). Nachdem die Blase definiert gefüllt ist, wird der Drei-Wege-Hahn so eingestellt, dass sowohl die Spritze als auch der Urinbeutel abgeklemmt sind und so das Schlauchsystem mit dem Blasenkatheter und Drucksensor **8** ein geschlossenes System bilden. In dieser Anordung misst der Sensor die Druckänderungen der Blase und zeigt damit die Aktivität des Detrusors. Vorteilhaft ist, zu beachten, dass der Drucksensor **8** an einen differentiellen Verstärkereingang angeschlossen werden kann, mit der wie oben angepassten Anschlussbox **7** für gleichzeitige Weiterleitung von EMG- und Druckmesssignalen kann dann das Drucksignal genauso aufgenommen werden wie das EMG-Signal.

Generell kann vorteilhaft das Signal für den Grundtonus (Druck) der Blase von den Messwerten nach erfolgreicher Stimulation abgezogen werden, um ein stärker auffallendes Signal zu erhalten.

### Referenzbeispiel: Intraoperative Verwendung

Die beispielhaft genannte, aber auch die in der allgemeinen Beschreibung genannte erfindungsgemäße Kontrollanordnung kann beispielsweise wie folgt in Methoden und Verwendungen eingesetzt werden:
Bei einer Operation kann mindestens eine handführbaren Stimulationssonde **19** im Operationsbereich vom Chirurgen so platziert werden, bis ein geeignetes Messsignal im Darstellungs- und Stimulierungsmodul **4** erkennbar wird. Dies zeigt an, dass ein geeigneter Nervenbereich gefunden wurde. An dieser Stelle kann dann für kontinuierliche Messung eine Sonde für kontinuierliche Stimulation **30** platziert werden. Für die Ableitung des EMG des Sphincter internus ani kann die bipolare EMG-Ableitelektrode **14** und/oder entsprechende Einzelelektroden einige mm seitlich der Öffnung des Anus eingeführt, beispielsweise in eine Tiefe von 10 bis 30 mm, beispielsweise an der 6-Uhr-Position. Der intravesikale Druck kann über den Drucksenson **8** ermittelt werden.

Beispielsweise kann die intermittierende Nervenstimulation mittels der handführbaren Stimulationssonde **19** zur Identifikation des autonomen Nervengewebes bei mesorektaler Exzision innerhalb definierter Operationsabschnitte verwendet werden. Sie kann so zur Festlegung der optimalen Stimulationsorte für die kontinuierliche Stimulierung (Stimulation) verwendet werden. Daneben kann der Nervenerhalt nach mesorektaler Exzision (oder die Verletzung von Nerven, was Entscheidungen über den weiteren Verlauf der Operation, wie beispielsweise über die Notwendigkeit des Anlegens eine künstlichen Darmausgangs bereits während der Operation, ermöglicht, intraoperativ ermittelt werden.

Beispielsweise kann während der Präparation am Kolon die intermittierende Nervenstimulation wie folgt eingesetzt werden: Während der Präparation des lymphovaskulären Stiels entlang der Rückseite der Arteria rectalis superior wird der präaortale Plexus hypogastricus superior und der Plexus mesentericus inferior auf der Aorta belassen und jeweils frühestmöglich mittels der handführbaren Stimulationssonde **19** stimuliert. Beispielsweise kann das über eine bestimmte Anzahl von Stimulationssalven (z.B. 3 Salven) erfolgen. Nach Absetzen der Arteria und vena mesenterica inferior erfolgt erneute Funktionskontrolle.

Bei mesorektaler Exzision kann mittels der intermittierenden Nervenstimulation nach lateraler Inzision des Beckenbodenperitoneums und Eröffnung des Spatium retrorektale die dorsale mesorektale Exzision beginnen und damit verbunden die Identifikation der Nervi hypogastrici durch intermittierende Neurostimulation mittels der handführbaren Stimulationssonde **19** mit Hilfe von einigen (z.B. 3) Stimulationssalven. Im Anschluss kann die Fortsetzung der dorsolateralen Präparation unter Schonung der nervalen Leitungsbahnen der Nn. hypogastrici erfolgen. Vor und nach Beginn der lateralen Dissektion kann intermittierend stimuliert werden, um zum frühestmöglichen Zeitpunkt parasympathische Nervenbahnen - Nervi splanchnici pelvici - zum Plexus hypogastricus inferior zu detektieren. Es erfolgen beidseitig jeweils mehrere (z.B. 10) Stimulationsssalven. Bei positiver Stimulationsantwort erfolgt vorteilhaft die Platzierung einer Sonde für kontinuierliche Stimulation **30**. Wird die Indikation zur PME (Partielle Mesorektale Exzision) intraoperativ bestätigt, kann kontinuierliche Stimulation bis zur Komplettierung der adäquaten mesorektalen Resektion und damit über die Darstellung der Messsignale die Funktion des Sphincter Ani und der Blase erfolgen. Bei Fortsetzung der lateralen Präparation können potentiell freiwerdende nervale Zuströme (Nn. splanchnici S2-S5) intermittierend auf aussagekräftige Stimulationsantworten überprüft werden. Hierzu kann die kontinuierliche Stimulation vorteilhaft unterbrochen werden. Ggf. erfolgt eine Umplatzierung der zur kontinuierlichen Stimulation verwendeten Elektrode.

Dann kann die anterolaterale mesorektale Präparation nach Inzision des peritonealen Umschlags oberhalb der Excavation rectorrinea bzw. rectovasalis folgen. Bei positiver kontinuierlicher Nervenstimulation kann während dieses Operationsabschnittes auf eine intermittierende Stimulation verzichtet werden.

Nach abgeschlossener PME bzw. TME (Partielle bzw. Totale Mesorektale Exzision) kann die intermittierende Nervenstimulation bei Rektumssektion nach Absetzen des Präparates erfolgen. Bei geplanter Exstirpation kann die intermittierende Nervenstimulation vor Beginn des perinealen Operationsabschnitts und kann nach vollständiger Exstirpation wiederholt werden.

Die kontinuierliche Nervenstimulation kann zur fortlaufenden Funktionsprüfung des autonomen Nervensystems im Beckenbereich bei lateraler und anterolateraler mesorektaler Exzision erfolgen.

Nach Identifikation der parasympathischen Leitungsbahnen (z.B. mittels der handführbaren Stimulationssonde **19**) wird die Sonde für kontinuierliche Stimulation **30** platziert und zur kontinuierlichen Nervenstimulation bei lateraler sowie anterolateraler Dissektion eingesetzt. Über einen Zeitraum von ca. 20 Minuten kann während der eigentlichen Präparationsphasen in beispielsweise 5 bis 20 Sekunden-Abschnitten disseziert und kontinuierlich stimuliert werden. Zwischen den Abschnitten können Pausen, beispielsweise von mindestens zehn Sekunden, eingehalten werden. Eine Umplatzierung einer Sonde für die kontinuierliche Stimulation **30** kann erfolgen, wenn mittels einer handführbaren Stimulationssonde **19** ein aussagekräftigerer Stimulationspunkt gefunden wird (besseres Signal).

Als Erfolgsparameter der intermittierenden und kontinuierlichen Prüfung werden messbare und reproduzierbare physiologische Antworten an den Erfolgsorganen Harnblase und Sphinkter ani (insbesondere internus) gewählt, wobei die vor- und nachstehend genannten Vorrichtungen, insbesondere die erfindungsgemäße Kontrollanordnung **1**, verwendet werden.

Für den perinealen Operationsabschnitt entfällt die Möglichkeit der EMG-Ableitung am Sphincter ani internus.

Indirekt können die Bestimmung der Stimulierbarkeit der Blase und des Sphincter ani auch Aussagen über mögliche Schädigungen der Sexualfunktion ermöglichen.

## Patentansprüche

1. Kontrollanordnung (1) zur im Wesentlichen gleichzeitigen Kontrolle der Stimulierbarkeit von durch das vegetative Nervensystem gesteuerten Funktionen im Beckenbereich, welche mindestens jeweils eine Stimulationselektrode (19, 30) und Sensoren zur Messung von Signalen aufweist, wobei die mindestens eine Stimulationselektrode (19, 30) zum Einbringen in den Beckenraum während einer Operation ausgeführt ist und die Kontrollanordnung (1) als Sensoren mindestens einen Sensor (8) zur Messung der Blasenfunktion und mindestens einen Sensor (14) zur Messung der Aktivität des Sphincter Ani internus aufweist und die Kontrollanordnung zur intraoperativen Kontrolle der besagten Stimulierbarkeit von durch das vegetative Nervensystem gesteuerten Funktionen im Beckenbereich eingerichtet ist, wobei der mindestens eine Sensor (8) zur Messung der Blasenfunktion jeweils solcher zur Messung des Blasendrucks ist und, als kennzeichnendes Merkmal, der oder die Sensoren zur Messung der Aktivität des Sphincter solche zur EMG-Messung sind, wobei die Aufnahme des EMG-Messignals durch einen software- und/oder hardware-implementierten Tiefpassfilter auf einen Bereich unter 30 Hz gefiltert wird.

2. Kontrollanordnung (1) nach Anspruch 1, **dadurch gekennzeichnet, dass** die Aufnahme des EMG-Messignals auf einen Bereich von 25 Hz oder niedriger gefiltert wird.

3. Kontrollanordnung nach Anspruch 1 oder Anspruch 2, **dadurch gekennzeichnet, dass** der oder die Sensoren (8) zur Messung der Blasenfunktion zur Messung des hydrostatischen Druckes in der Blase ausgelegt sind und mit einem Blasenkatheter (25) verbunden sein kann bzw. können.

4. Kontrollanordnung nach einem der Ansprüche 1 oder 2 oder 3, wobei der oder die Sensoren zur Messung der Aktivität des Sphincter als wenigstens eine oder mehrere bipolare EMG-Ableitelektrode (14) ausgestaltet ist.

5. Kontrollanordnung nach einem der Ansprüche 1 bis 4, **dadurch gekennzeichnet, dass** die Sensoren (8, 14) und die Stimulationselektrode(n) (19, 30) mit einem Datenerfassungs- und Stimulierungsmodul (4) verbunden sind, welches neben einer Anzeigevorrichtung und optionalen beispielsweise computer- und softwaregestützten Auswertungs- und Dokumentationssausstattungen auch eingerichtet ist zur Aufarbeitung der Messsignale und außerdem zur Beaufschlagung der Stimulationselektrode(n) (19, 30) mit Stimulationspulsen ausgestattet ist.

6. Kontrollanordnung nach einem der Ansprüche 1 bis 5, **dadurch gekennzeichnet, dass** die mindestens eine Stimulationselektrode eine handführbare Stimulationssonde (19) und/oder eine Sonde für kontinuierliche Stimulation (30), oder eine Kombination davon, darstellt.

7. Kontrollanordnung nach einem der Ansprüche 1 bis 6, **dadurch gekennzeichnet, dass** sie außerdem eine Anschlussbox (7) und/oder Druckmessbox aufweist, welche die Signale der Sensoren (8, 14) aufnimmt und an ein bzw. das Datenerfassungs- und Stimulierungsmodul (4) weiterleitet.

8. Kontrollanordnung nach einem der Ansprüche 1 bis 7, **dadurch gekennzeichnet, dass** die Stimulationssignale und die EMG-Messsignale und/oder der als Sensor (14) für die Messung der Aktivität des Sphincter verwendeten EMG-Ableitelektrode(n) aufeinander abgestimmt sind, um eine gegenseitige Störung zu verhindern, indem das EMG-Messsignal bei Stimulation des glatten Muskels in einem Bereich unter 30 Hz durch entsprechende Filterung mittels eines Tiefpassfilters gefiltert wird.

9. Kontrollanordnung nach einem der Ansprüche 1 bis 8, **dadurch gekennzeichnet, dass** sie eingerichtet ist zur intraoperativen Kontrolle der Funktionsfähigkeit des Nervensystems im Beckenbereich eines Patienten, wobei ein oder mehrere Stimulationselektroden (19, 30), ein oder mehrere Sensoren (8) für die Messung der Blasenfunktion und ein oder mehrere Sensoren (14) zum Einsatz zur Messung der Aktivität des Sphincter Ani eingerichtet sind und zur Prüfung, ob die Stimulation zu Messsignalen führt, die zeigen, dass die Nervenverbindung zum entsprechenden Erfolgsorgan Blase und/oder Sphincter Ani intakt oder ganz oder teilweise geschädigt ist.

## Claims

1. Monitoring arrangement (1) for substantially simultaneous monitoring of the stimulation capability of functions in the pelvic region which are controlled by the autonomic nervous system, which has at least one stimulation electrode (19, 30) in each case and sensors for measurement of signals, wherein the at least one stimulation electrode (19, 30) is designed for insertion into the pelvic cavity during an operation and the monitoring arrangement (1) comprises, as sensors, at least one sensor (8) for measuring the bladder function and at least one sensor (14) for measuring the activity of the internal anal sphincter and the monitoring arrangement is arranged for intra-operative monitoring of said stimulation capability of functions in the pelvic region which are controlled by the autonomic nervous system, wherein the at least one sensor (8) for measuring the bladder function is in each case a sensor for measuring the bladder pressure and, as a characterising feature, the sensor or sensors for measuring the activity of the sphincter are sensors for EMG measurement, wherein the reception of the EMG measurement signal is filtered by a software-operated and/or hardware-operated low pass filter to a range below 30 Hz.

2. Monitoring arrangement (1) as claimed in claim 1, **characterised in that** the reception of the EMG measurement signal is filtered to a range of 25 Hz or lower.

3. Monitoring arrangement as claimed in claim 1 or claim 2, **characterised in that** the sensor or sensors (8) for measuring the bladder function are arranged for measuring the hydrostatic pressure in the bladder and can be connected to a bladder catheter (25).

4. Monitoring arrangement as claimed in any one of claims 1 or 2 or 3, wherein the sensor or sensors for measuring the activity of the sphincter are designed as at least one or a plurality of bipolar EMG lead electrodes (14).

5. Monitoring arrangement as claimed in any one of claims 1 to 4, **characterised in that** the sensors (8, 14) and the stimulation electrode(s) (19, 30) are connected to a data capture and stimulation module (4) which, in addition to a display device and optional evaluation and documentation facilities, which are e.g. computer-aided and software-aided, is also arranged to process the measurement signals and is also equipped to apply stimulation pulses to the stimulation electrode(s) (19, 30).

6. Monitoring arrangement as claimed in any one of claims 1 to 5, **characterised in that** the at least one stimulation electrode is a manually guidable stimulation probe (19) and/or a probe for continuous stimulation (30), or a combination thereof.

7. Monitoring arrangement as claimed in any one of claims 1 to 6, **characterised in that** it also comprises an attachment box (7) and/or pressure measuring box which receives the signals of the sensors (8, 14) and passes them to a or the data capture and stimulation module (4).

8. Monitoring arrangement as claimed in any one of claims 1 to 7, **characterised in that** the stimulation signals and the EMG measurement signals and/or the EMG lead electrode(s), which is/are used as a sensor (14) for measuring the activity of the sphincter, are tuned to one another in order to prevent mutual interference **in that** the EMG measurement signal is filtered upon stimulation of the smooth muscle in a range below 30 Hz by corresponding filtration by means of a low pass filter.

9. Monitoring arrangement as claimed in any one of claims 1 to 8, **characterised in that** it is arranged for intra-operative monitoring of the functionality of the nervous system in the pelvic region of a patient, wherein one or a plurality of stimulation electrodes (19, 30), one or a plurality of sensors (8) are arranged for measurement of the bladder function, and one or a plurality of sensors (14) are arranged for use in measuring the activity of the internal anal sphincter, and for checking whether the stimulation leads to measurement signals which show that the nerve connection to the corresponding effector organ, bladder and/or internal anal sphincter is intact or is wholly or partially damaged.

## Revendications

1. Agencement de contrôle (1) pour le contrôle essentiellement simultané de la capacité à être stimulées de fonctions commandées par le système nerveux végétatif dans le bassin, lequel présente à chaque fois au moins une électrode de stimulation (19, 30) et des capteurs de mesure de signaux, sachant que l'au moins une électrode de stimulation (19, 30) est conçue pour être introduite dans l'espace pelvien pendant une opération et l'agencement de contrôle (1) présente, en tant que capteurs, au moins un capteur (8) pour mesurer le fonctionnement de la vessie et au moins un capteur (14) pour mesurer l'activité du sphincter anal interne et l'agencement de contrôle est prévu pour le contrôle intraopératoire de ladite capacité à être stimulées de fonctions commandées par le système nerveux végétatif dans le bassin, sachant que l'au moins un capteur (8) pour mesurer le fonctionnement de la vessie est à chaque fois un de ceux pour mesurer la pression de la vessie et, en tant qu'attribut caractérisant, le ou les capteur(s) pour mesurer l'activité du sphincter est/sont de ceux pour la mesure EMG, sachant que la réception du signal de mesure EMG est filtrée par un filtre passe-bas sur une plage inférieure à 30 Hz implémenté par un logiciel et/ou un hardware.

2. Agencement de contrôle (1) selon la revendication 1, **caractérisé en ce que** la réception du signal de mesure EMG est filtrée sur une plage de 25 Hz ou moins.

3. Agencement de contrôle selon la revendication 1 ou 2, **caractérisé en ce que** le ou les capteur(s) (8) pour mesurer le fonctionnement de la vessie est/sont conçu(s) pour mesurer la pression hydrostatique dans la vessie et peut/peuvent être relié(s) à un cathéter de vessie (25).

4. Agencement de contrôle selon la revendication 1 ou 2 ou 3, dans lequel le ou les capteur(s) pour mesurer l'activité du sphincter a/ont la forme d'au moins une ou plusieurs électrode(s) de dérivation EMG bipolaire(s) (14).

5. Agencement de contrôle selon l'une des revendications 1 à 4, **caractérisé en ce que** les capteurs (8, 14) et la/les électrode(s) de stimulation (19, 30) sont reliés à un module de saisie de données et de stimulation (4), lequel est également prévu, outre un dispositif d'affichage et des équipements en option d'évaluation et de documentation, par exemple assistés par ordinateur et par logiciel, pour traiter les signaux de mesure et en outre, pour alimenter la/les électrode(s) de stimulation (19, 30), est doté d'impulsions de stimulation.

6. Agencement de contrôle selon l'une des revendications 1 à 5, **caractérisé en ce que** l'au moins une électrode de stimulation représente une sonde de stimulation (19) pouvant être dirigée à la main et/ou une sonde pour une stimulation continue (30), ou une combinaison de celles-ci.

7. Agencement de contrôle selon l'une des revendications 1 à 6, **caractérisé en ce qu'**il présente en outre un boîtier de connexion (7) et/ou un boîtier de mesure de pression, qui reçoi(ven)t les signaux des capteurs (8, 14) et les transmet(tent) à un, respectivement au, module de saisie de données et de stimulation (4).

8. Agencement de contrôle selon l'une des revendications 1 à 7, **caractérisé en ce que** les signaux de stimulation et les signaux de mesure EMG et/ou la/les électrode(s) de dérivation EMG employée (s) en tant que capteur(s)(14) pour mesurer l'activité du sphincter sont coordonnés entre eux pour éviter une perturbation réciproque, **en ce que** le signal de mesure EMG lors de la stimulation du muscle lisse est filtré dans une plage inférieure à 30 Hz par une filtration correspondante via un filtre passe-bas.

9. Agencement de contrôle selon l'une des revendications 1 à 8, **caractérisé en ce qu'**il est prévu pour le contrôle intraopératoire de la capacité à fonctionner du système nerveux dans le bassin d'un patient, sachant qu'une ou plusieurs électrode (s) de stimulation (19, 30), un ou plusieurs capteur(s) (8) pour mesurer le fonctionnement de la vessie et un ou plusieurs capteur(s) (14) destiné(s) à être utilisé(s) pour mesurer l'activité du sphincter anal sont prévus, et pour vérifier si la stimulation conduit à des signaux de mesure qui indiquent que la liaison nerveuse vers l'organe effecteur correspondant, la vessie et/ou le sphincter anal, est intacte ou totalement ou partiellement endommagée.
